Europäisches Patentamt

(19) European Patent Office

Office européen des brevets

(11) Publication number: **0 151 941**
**A2**

(12) # EUROPEAN PATENT APPLICATION

(21) Application number: 85100322.8

(22) Date of filing: 15.01.85

(51) Int. Cl.⁴: **C 07 D 301/12**
C 07 D 303/14

(30) Priority: 31.01.84 US 575507

(43) Date of publication of application:
21.08.85 Bulletin 85/34

(84) Designated Contracting States:
BE CH DE FR GB IT LI NL

(71) Applicant: Union Camp Corporation
1600 Valley Road
Wayne New Jersey 07470(US)

(72) Inventor: McElligott, Lois T.
1158 Wheatsheaf Lane
Abington Pennsylvania 19001(US)

(74) Representative: von Füner, Alexander, Dr. et al,
Patentanwälte Schiff, von Füner Strehl, Schübel-Hopf,
Ebbinghaus, Finck Mariahilfplatz 2 u. 3
D-8000 München 90(DE)

(54) Epoxidation of olefinic alcohols.

(57) A method is disclosed for the epoxidation of water-insoluble olefinic alcohols, by reaction with hydrogen peroxide in the presence of an epoxidation catalyst and a phase transfer catalyst.

Croydon Printing Company Ltd.

EPOXIDATION OF OLEFINIC ALCOHOLS

BACKGROUND OF THE INVENTION

Field of the Invention

The invention relates to a method for the epoxidation of olefinic alcohols and more particularly relates to a method for the epoxidation of water-insoluble olefinic alcohols.

Brief Description of the Prior Art

A method for the epoxidation of olefin hydrocarbons was described by Venturello et al. in the J. Org. Chem., 1983, 48, 3831-33.

The present invention provides a method which advantageously may use relatively low concentrations of epoxidation agent and provides advantageous yields of desired product.

SUMMARY OF THE INVENTION

The invention comprises a method for the epoxidation of water-insoluble olefinic alcohols which comprises:

- 2 -                                              0151941

providing the alcohol in a liquid state;

providing an aqueous solution of hydrogen peroxide;

bringing the liquid state alcohol together with the solution of hydrogen peroxide, whereby there is obtained a heterogeneous reaction mixture having an aqueous phase and an organic phase, in the presence of a catalytic proportion of an epoxidation catalyst and a phase transfer catalyst which functions between the phases;

said aqueous phase having a pH within the range of from about 4 to about 6.


## DETAILED DESCRIPTION OF THE PREFERRED EMBODIMENTS OF THE INVENTION

The term "water-insoluble olefinic alcohol" as used herein means an olefinic alcohol having relatively little solubility in water at 25°C., i.e., less than about 5 percent. Advantageously the method of the invention is employed to epoxidize alcohols having 6 to 30 carbon atoms, inclusive. Representative of such alcohols are alpha-terpineol, terpinen-4-ol, geraniol, linalool, cinnamyl alcohol, oleyl alcohol, methyl heptenol, cholesterol and the like.

In the method of the invention, the alcohol is provided in a liquid form. Solutions of the alcohol in an inert organic solvent are advantageous to provide the alcohol reactant. By the term "inert organic solvent" we mean a solvent which is insoluble in water and does not enter into or otherwise adversely affect the desired course of the epoxidation reaction. Representative of preferred inert organic solvents are water insoluble hydrocarbon solvents such as benzene, toluene, naphtha, n-hexane and the like.

Another advantage associated with the method of the invention is that the reactant alcohols, by their nature, enhance the polarity of the organic solution by the presence of the hydroxyl group. Olefin hydrocarbons, without the hydroxyl group, need a more polar solvent such as dichloroethane in order to give good yields. Therefore as long as the particular olefinic alcohol is not too large so as to give it more hydrocarbon-like character, a wide range of hydrocarbon solvents can be used in addition to the less desirable chlorinated solvents such as dichloroethane.

The epoxidation agent is an aqueous solution of hydrogen peroxide, which solution is immiscible with the organic alcohol in liquid form. The concentration of the hydrogen peroxide solution is not a critical factor. Advantageously, a 2 to 50 percent concentration solution is employed.

To initiate the epoxidation, the two reactants (alcohol and epoxidation agent) are brought together to form a two-phase reaction mixture. A catalytic proportion of an epoxidation catalyst is present in the aqueous phase.

Preferred as the epoxidation catalyst is the two-component association consisting of tungstate and phosphate or arsenate ions described in Venturello et al., supra. A catalytic proportion is generally within the range of from about 1 percent to about 10 percent by weight of the hydrogen peroxide solution.

A phase transfer catalyst is also present in the reaction mixture to promote the desired epoxidation.

Phase transfer catalysts are generally well-known in the art as is their preparation and include quaternary salts and quaternary resins where the central atom is nitrogen, phosphorus, arsenic, bismuth, antimony and the like; amine salts, ammonium salts, crown ethers, polyethers, cryptands, phosphonium salts, arsonium salts, antimonium salts, bismuthonium salts, alpha-phosphorylsulfoxides, sulfones, sulfides and the like.

Representative of commonly employed phase transfer catalysts are those described in U.S. Patent 3,992,432; the disclosure of which is hereby incorporated by reference. Preferred as the phase transfer catalyst is a mixture of trialkylmethylammonium chlorides where alkyl is $C_8$-$C_{10}$. Such mixtures are commercially available (Adogen 464, Ashland Chemical Company).

The phase transfer catalyst is employed in a catalytic proportion, i.e., generally within the range of from about 0.001 to 5.0 percent by weight of the reaction mixture; preferably 2.0 to 4.0 percent.

Critical to the method of the invention is the maintenance of a pH in the aqueous phase of the reaction mixture within the range of from about 4 to about 6; preferably 4.8 to 5.2 and most preferably 5.0. The desired pH can be obtained by addition of appropriate proportions of base to reaction mixture, such as sodium hydroxide, triethylamine and the like.

The method of the invention may be carried out over a wide range of temperatures, advantageously from about room temperature to 75°C. A wide range of pressure conditions may also be employed, i.e., sub-atmospheric to superatmospheric, preferably atmospheric.

In the preferred embodiment method of the invention, the ratio of reactants (alcohol to peroxide) on a molar basis is generally within the range of from 1:0.5 to 1:2; preferably 1:1.

The following examples describe the manner and process of making and using the invention and set forth the best mode contemplated by the inventor of carrying out the invention but are not to be construed as limiting. All parts are by weight unless otherwise indicated.

EXAMPLE 1

A reactor vessel was charged with 0.55g sodium tungstate dihydrate, 0.82g 40% phosphoric acid and 17.0g 8%

- 6 -                    0151941

hydrogen peroxide. The pH was adjusted to 4.0 with 10% sodium hydroxide. A mixture of 10.9g terpinen-4-ol (95%) 0.27g of Adogen 464, supra., and 5 ml toluene was added to the peroxide solution. After a gradual exotherm to 64°C the mixture was heated manually to 70°C and held for 30 minutes. After cooling, the aqueous layer was removed and the organics were washed with water. The product was analyzed by gas chromatography. Based on available peroxide, the yield was 75% cis-terpinen-4-ol epoxide and 6% trans-terpinen-4-ol epoxide with 100% conversion of terpinen-4-ol.

EXAMPLE 2

A reactor vessel was charged with 10.9g terpinen-4-ol (95%) 0.27g Adogen 464, supra., and 5 ml cyclohexane. A solution of 0.55g sodium tungstate dihydrate, 0.82g 40% phosphoric acid and 34.2g 8% hydrogen peroxide was prepared. The pH was adjusted to 5.0 with 10% sodium hydroxide. The reactor was heated to 40°C and the peroxide solution was added dropwise. The temperature was controlled to 70°C with a water bath. After the exotherm subsided, the reactor was heated manually to 70°C and held for 1 hour. After cooling the aqueous layer was removed and the organics were washed with water. The product was analyzed by gas chromatography. The yield was 74% cis-terpinen-4-ol epoxide and 3% trans-terpinen-4-ol epoxide with a 92% conversion of terpinen-4-ol.

EXAMPLES 3 & 4

Procedure

A reactor vessel was charged with 0.83g sodium tungstate dihydrate, 1.53g 40% phosphoric acid and 25.5g of 8% hydrogen peroxide. The solution was adjusted to pH 4.0 with 10% sodium hydroxide. A mixture of alcohol, in an amount indicated below, 0.41g Adogen 464, supra., and 7.5 ml dichloroethane was added to the peroxide solution. After the exotherm was controlled to 70°C in a water bath, the reactor was maintained at 70°C for 1 hour with manual heating. After cooling, the aqueous layer was removed and the organics were washed with water.

The yields and conversions, based on available peroxide, are listed in Table 1, below.

TABLE 1

| Feed | % Conversion | % Yield |
| --- | --- | --- |
| 15.5g Geraniol (99%) | 90 | 64 - Geraniol epoxide |
| 15.9g Linalool (97%) | 60 | 37 - Linalool oxide<br>13 - 6-ethenyl-2-hydroxy 6,2,2-trimethylpyran |

WHAT IS CLAIMED:

1. A method for the epoxidation of water-insoluble olefinic alcohols, which comprises:

providing the alcohol in a liquid state;

providing an aqueous solution of hydrogen peroxide;

bringing the liquid state alcohol together with the solution of hydrogen peroxide, whereby there is obtained a heterogeneous reaction mixture having an aqueous phase and an organic phase, in the presence of a catalytic proportion of an epoxidation catalyst and a phase transfer catalyst which functions between the phases;

said aqueous phase having a pH within the range of from about 4 to about 6.

2. The process of claim 1 wherein the alcohol is selected from the group consisting of terpinen-4-ol, alpha-terpineol, beta-terpineol, nerol, geraniol, linalool and nopol.